# EUROPÄISCHE PATENTSCHRIFT

(11) **EP 0 684 036 B1**
(45) Veröffentlichungstag und Bekanntmachung des Hinweises auf die Patenterteilung: **23.08.2000**
(21) Anmeldenummer: 95104370.2
(22) Anmeldetag: 24.03.1995
(51) Int. Cl.: A61K 7/135, A61K 7/13

(54) **Verwendung von Polyorganosiloxanen in Mitteln zum Blondieren von menschlichen Haaren**
Use of polyorganosiloxanes in compositions for bleaching of human hair
Utilisation des polyorganosiloxanes dans les compositions pour blanchir des cheveux humains

(30) Priorität: 27.05.1994 DE 4418548
(43) Veröffentlichungstag der Anmeldung: 29.11.1995
(73) Patentinhaber: GOLDWELL GmbH, D-64297 Darmstadt (DE)
(72) Erfinder: Lorenz, Heribert, D-64401 Gross-Bieberau (DE); Eberling, Walter, D-64560 Riedstadt (DE)

(56) Entgegenhaltungen:
- DE-A- 4 425 096
- FR-A- 2 696 930
- GB-A- 2 188 948
- PATENT ABSTRACTS OF JAPAN vol. 13 no. 158 (C-586) ,17.April 1989 & JP-A-63 313717 (LION CORP.) 21.Dezember 1988,
- PATENT ABSTRACTS OF JAPAN vol. 12 no. 365 (C-532) ,29.September 1988 & JP-A-63 119414 (SHISEIDO CO. LTD.) 24.Mai 1988,

## Beschreibung

Die vorliegende Erfindung betrifft die Verwendung von Polyorganosiloxanen in pulverförmigen Mitteln zum Blondieren von menschlichen Haaren mit verbesserter Aufhellungswirkung.

Herkömmliche Mittel zum Blondieren bzw. Bleichen von menschlichen Haaren bestehen aus mindestens einem festen Peroxid, insbesondere einem Persulfat, und einem pulverförmigen Trägermaterial. Dieses Pulver wird bei Gebrauch mit einer 6- bis 12-prozentigen Wasserstoffperoxid-Lösung angerührt und auf das Haar aufgebracht. Beispiele für derartige Zusammensetzungen finden sich in der einschlägigen Fachliteratur, beispielsweise bei K. Schrader, Grundlagen und Rezepturen der Kosmetika, 2. Aufl. (1989, Hüthig Buchverlag), S. 815 bis 823.

Die Blondiereigenschaften dieser Blondierpulver sind jedoch bisher nicht immer befriedigend. Es bestand daher ein Bedürfnis, Blondierpulver mit verbesserter Aufhellungswirkung zu entwickeln.

Es wurde nunmehr gefunden, daß ein pulverförmiges Mittel zum Blondieren von menschlichen Haaren mit verbesserter Aufhellungs- und damit Blondierwirkung erhalten wird, wenn man dem mindestens eine aufhellend wirkende, d.h. bleichaktive Substanz, insbesondere eine feste Per-Verbindung, enthaltenden Mittel etwa 0,01 bis etwa 2,5 Gew.-%, vorzugsweise 0,05 bis 1 Gew.-%, berechnet auf die Gesamtzusammensetzung des Pulvers, mindestens eines Polyorganosiloxans zusetzt.

Das so zusammengesetzte Blondiermittel-Pulver ergibt nach dem Anrühren mit einer Wasserstoffperoxid-Lösung auf dem Haar eine exzellente, gegenüber üblichen Blondierpulvern deutlich verbesserte Blondierwirkung.

Darüber hinaus wird auch eine verbesserte Stabilität und Handhabbarkeit des anwendungsfertigen Gemisches aus Pulver und Wasserstoffperoxid-Lösung erzielt.

Geeignete Polyorganosiloxane sind insbesondere die bekannten Polydimethylsiloxane, wie sie unter dem Trivialnamen "Dimethicone" und dessen Derivaten geläufig sind (vgl. CTFA-International Cosmetic Ingredient Dictionary, 4th Ed., S. 220 - 223), entsprechend der Strukturformel: 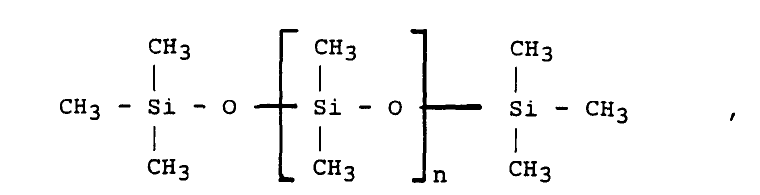 oder Dimethicone-Copolyole der Formel 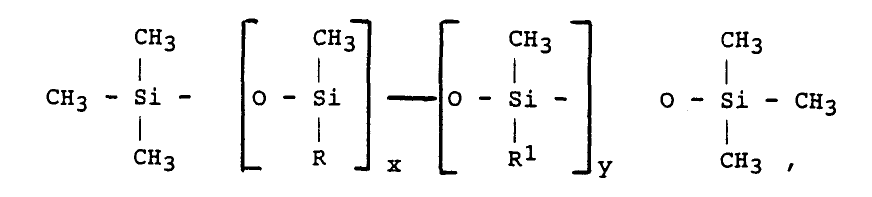 worin R eine Alkylgruppe mit 1 bis 20 Kohlenstoffatomen, R¹ eine Polyethergruppe mit 1 bis 60 Ethylenoxid- und/oder Propylenoxid-Einheiten, und x und y eine Zahl von 1 bis 500 bedeuten.

Ein erfindungsgemäß zum Einsatz gelangendes Alkylmethicone-Copolyol weist vorzugsweise ein Molgewicht von etwa 10 000 bis etwa 40 000, vorzugsweise etwa 22 000 bis etwa 32 000, auf.

Die Alkylgruppe R ist vorzugsweise ein Laurylrest, ein C₁₂-C₁₄-Alkylrest, ein Cetylrest, ein C₈-Alkylrest oder ein C₁₈-Alkylrest, jedoch sind auch breite Alkylgruppenverteilungen durchaus geeignet.

Die Polyol-Substitution liegt vorzugsweise bei 1, 3, 5, 10 bzw. 25 Mol.-%; der Ethylen- bzw. Propylenoxid-Anteil im Polyether bei 12 EO, 18 EO/18 PO bzw. 24 EO/24 PO.

x ist vorzugsweise eine Zahl zwischen 60 und 245; y zwischen 1 bis 25; die Gesamtzahl der Siloxan-Einheiten beträgt vorzugsweise 60, 90, 145, 200 bzw. 245.

Bevorzugt ist Cetyl- oder Laurylmethicone-Copolyol.

Ein entsprechendes Produkt wird von der Dow Corning Co. unter der Bezeichnung "Dow Corning Q2-5200" vertrieben.

Ein weiteres bevorzugtes alkylmodifiziertes Methicone-Copolyol ist ein solches, das einen Polyetherrest R¹

-(CH₂)ₙ - O - (C₂H₄O)ₘ - (C₃H₆O)₂ X

besitzt, wobei n 0 bis 5 und die Summe aus m + z 1 bis 60 beträgt, und X vorzugsweise H oder auch eine C₁-C₄-Alkylgruppe bedeutet. R stellt dann eine Alkylgruppe mit 14 bis 16 Kohlenstoffatomen dar.

Ebenfalls im Rahmen der Erfindung geeignet sind Dimethicone-Copolyole, wie sie beispielsweise von den Firmen Dow Corning Co., Goldschmidt, Union Carbide und Shin Etsu vertrieben werden, wobei x eine Zahl von 5 bis 15, y eine Zahl von 3 bis 7 und R¹ eine Gruppe (CH₂)ₘ-O-(A-O)ₙ-X bezeichnen, wobei m 2 oder 3, n eine Zahl von 10 bis 15, A eine Propylen- oder Ethylengruppe und X H oder eine C₁-C₄-Alkylgruppe bedeuten.

Diese Produkte sind unter den Handelsnamen "Abil B 8842, B 8843, B 8847, B 8851, B 8852, B 8863, B 8873, B 88183" und B 88184" (Goldschmidt), "Dow Corning 190, 193 bzw. Q2-5220 Polyether", "Shin Etsu KF 351A, KF 352A, KF 353A, KF 354A, KF 355A, KF 615A, KF 625A bzw. KF 954A" sowie "Silwet" (Union Carbide) auf dem Markt erhältlich.

Bei dem Rest R handelt es sich dabei vorzugsweise um eine Methyl-, Lauryl- oder Cetylgruppe.

Weitere geeignete Polysiloxane sind aminomodifizierte Silicone, wie sie unter der Standardbezeichnung "Amodimethicone" bekannt sind.

Bevorzugt geeignete Polysiloxane weisen ein Molekulargewicht zwischen etwa 1000 und etwa 200 000, vorzugsweise von etwa 3000 bis etwa 100 000, insbesondere etwa 5000 bis etwa 75 000, auf. Sie sind beispielsweise in Römpp Chemie Lexikon, 9.Aufl., Bd. 5, S. 4168 - 4169, und Kirk Othmer, Encyclopedia of Chemical Technology, 3rd Ed., Vol. 20, S. 936 ff., sowie in der GB-A 2 178 443, der EP-A 181 773 und der EP-A 400 976 definiert.

Der Zusatz des Polyorganosiloxans erfolgt, wenn es sich um einfache Pulverzusammensetzungen der beispielsweise bei Schrader, l.c., beschriebenen Art handelt, durch einfaches Vermischen.

Gemäß einer bevorzugten Ausführungsform der Erfindung werden die Blondierpulver jedoch als staubfreie Produkte verwendet, die als Granulate bzw. Agglomerate vorliegen.

Falls es sich dabei beispielsweise um die in der EP-A 560 088 beschriebenen Produkte, die ein Öl bzw. ein flüssiges Wachs enthalten, handelt, erfolgt das Aufbringen des Polyorganosiloxans vorzugsweise gleichzeitig mit diesem Öl oder Wachs, zweckmäßigerweise durch Aufsprühen.

Das Polyorganosiloxan kann jedoch auch im Ausgangspulver enthalten sein, beispielsweise im Gemisch mit Siliciumdioxid oder dem Parfum.

Handelt es sich hingegen um staubfreie Blondierpulver, die durch Agglomerieren der pulverförmigen Ausgangsbestandteile mittels Aufsprühen von geschmolzenen Wachsen oder C₁₀-C₁₈-Fettsäurealkanolamiden oder Verschmelzen mit denselben bei erhöhter Temperatur hergestellt wurden, so können die Polyorganosiloxane mit diesen aufgebracht oder auch dem pulverförmigen Ausgangsprodukt zugesetzt sein.

Das erfindungsgemäße Mittel zum Blondieren bzw. Bleichen von menschlichen Haaren enthält die in solchen Mitteln bekannten und üblichen Bestandteile, es wird hierzu, zur Vermeidung von Wiederholungen, auf Schrader, l.c., verwiesen.

Geeignete aufhellend wirkende, bleichaktive Substanzen sind Peroxide, vorzugsweise Alkalipersulfate wie Kalium- und Ammoniumpersulfat, Magnesiumperoxid, Harnstoffperoxid, Melaminperoxid, etc. sowie Gemische derselben.

Es können jedoch auch Zusammensetzungen verwendet werden, die kein oder nur geringe Mengen Persulfat und statt dessen andere aufhellende Bestandteile, beispielsweise Ammoniumcarbonat, Ammoniumbicarbonat, Ammoniumcarbamat, Ammoniumchlorid, Ammoniumsulfat, Ammoniumphosphate, Ammoniumbromid, Ammoniumjodid, Ammoniumnitrat, Ammoniumthiosulfat, Ammoniummolybdat, Ammoniumvanadat, Ammoniumsulfamat, Ammoniumcitrat, Ammoniumsalicylat, Ammoniumvalerat, Ammoniumtartrat, Ammoniumbenzoat, Ammoniumacetat, Ammoniumformiat und/oder Ammoniumlactat enthalten.
Die Teilchengrößen der erfindungsgemäßen Blondiermittel liegen in der Regel unterhalb 1 mm, vorzugsweise unterhalb 500 Mikron, beispielsweise bei weniger als 400 Mikron, was eine ausgezeichnete Verarbeitbarkeit, d. h. Mischbarkeit mit Wasser oder einer wäßrigen Wasserstoffperoxid-Lösung vor Applikation auf das menschliche Haar gewährleistet.

Die Anwendung des Mittels geschieht in an sich bekannter und üblicher Weise:

Durch Vermischen des pulverförmigen Blondiermittels mit einer 6- bis 12-prozentigen Wasserstoffperoxid-Lösung, wobei etwa 1 Teil des Pulvers mit etwa 1,5 Teilen der Peroxid-Lösung, vorzugsweise 9%iger H₂O₂-Lösung, homogen vermischt und anschließend etwa 20 bis etwa 60 Minuten, je nach Temperatur, auf das Haar zur Einwirkung gebracht wird.

Die folgenden Beispiele dienen der Illustration der Erfindung.

### Beispiel 1

| | |
|---|---|
| Siliciumdioxid (Diatomeenerde) | 3,20(Gew.-%) |
| Siliciumdioxid (pyrogenes SiO₂) | 5,20 |
| Natriumcarboxymethylcellulose | 3,50 |
| Harnstoff | 2,00 |
| Natriumlauroylsarkosinat | 0,80 |
| Natriumstearat | 1,20 |
| Natriumcarbonat | 1,00 |
| Natriummetasilikat | 6,00 |
| Stärkepulver | 3,50 |
| Kaliumpersulfat | 58,00 |
| Magnesiumperoxid | 4,00 |
| Paraffinöl (Paraffinum perliquidum, DAB 9) | 11,50 |
| Polydimethylsiloxan (Dimethicone) | 0,10 |

Es wird ein staubfreies Pulver erhalten, das mit einer bekannten 9%igen H₂O₂-Lösung im Gewichtsverhältnis 1 : 1,5 gut angerührt werden kann und eine gegenüber einer identischen, jedoch kein Dimethylpolysiloxan enthaltenden Zusammensetzung eine sichtbar verstärkte Aufhellung des Haares ergibt.

99% der Teilchen haben einen Durchmesser von < 400 Mikron.

Die Herstellung des Pulvers erfolgte durch Aufsprühen des Paraffinöl/Polydimethylsiloxan-Gemischs auf die Pulvergrundmasse bei etwa 20 °C im Wirbelsprühverfahren.

### Beispiel 2

| | **A** | **B** | **C** |
|---|---|---|---|
| Siliciumdioxid (Kieselgur) | 1,2 | 1,7 | 2,2 |
| Hydroxyethylcellulose | 1,4 | 1,4 | 1,4 |
| Carboxymethylcellulose | 2,5 | 2,5 | 2,5 |
| Natriumlaurylsulfat | 2,5 | 2,5 | 2,5 |
| Eiweißhydrolysat | 0,5 | 0,5 | 0,5 |
| Natriumstearat | 1,0 | 1,0 | 1,0 |
| Stärke | 1,0 | 1,0 | 1,0 |
| Natriumcarbonat | 1,1 | 1,1 | 1,1 |
| Ultramarinblau | 0,2 | 0,2 | 0,2 |
| Magnesiumperoxid | 4,0 | 4,0 | 4,0 |
| Natriummetasilikat | 9,0 | 9,0 | 9,0 |
| Kaliumpersulfat | 58,0 | 58,0 | 58,0 |
| Polyvinylpyrrolidon | 4,0 | 4,0 | 4,0 |
| Natriumalginat | 0,4 | 0,4 | 0,4 |
| Kokosmonoethanolamid | 10,0 | 10,0 | 10,0 |
| Oleamide | 2,0 | 2,0 | 2,0 |
| Parfumöl | 0,5 | 0,5 | 0,5 |
| Polyorganosiloxan (Dimethicone-Copolyol) | 1,0 | - | - |
| Polyorganosiloxan (Dimethicone) | - | 0,5 | - |

Die Herstellung erfolgte nach dem in der DE 43 34 183 beschriebenen Verfahren auf die Weise, daß zunächst in einem Wirbelschichtreaktor alle Bestandteile außer Kokosmonoethanolamid, Oleamide und Polyorganosiloxan auf 70 bis 75°C aufgeheizt wurden.

Anschließend wurden die auf etwa 40 °C erwärmten Gemische aus Kokosmonoethanolamid, Oleamide und Polyorganosiloxan auf die erhitzte Pulvermischung aufgesprüht, im Luftstrom auf etwa 30 °C abgekühlt und das erhaltene Agglomerat getrocknet.

Die erhaltenen Agglomerate wurden im Verhältnis 1 : 1,5 mit 9%iger H₂O₂-Lösung angerührt und auf menschliche Haarsträhnen aufgebracht.

Nach etwa 40-minütiger Einwirkung wurden die Haarsträhnen gespült und der Aufhellungsgrad der mit den Formulierungen 2A, 2B und 2C behandelten Haare verglichen.

| Produkt | "L∗" |
|---|---|
| 2A | 58,42 |
| 2B | 57,58 |
| 2C | 51,63 |

Die Bestimmung des Wertes "L∗" erfolgte mit dem Farbmeßgerät "Cromameter CR 200" der Fa. Minolta.

L* ist ein Maß für die Helligkeit in Prozent gegenüber einer völlig weißen Oberfläche, deren Wert mit 100% angesetzt ist. Das bedeutet, je höher der Wert "L*" ist, desto stärker die Aufhellung.
Diese Ergebnisse zeigen, daß mit den erfindungsgemäßen Zusammensetzungen 2A und 2B gegenüber der nicht erfindungsgemäßen Zusammensetzung 2C eine signifikant bessere Aufhellung erhielt wurde.

### Beispiel 3

| | **A** | **B** | **C** | **D** | **E** |
|---|---|---|---|---|---|
| Siliciumdioxid (Kieselgur) | 24,5 | 25,0 | 24,5 | 25,0 | (Gew.-%) 25,5 |
| Natriumalginat | 7,5 | 7,5 | 7,5 | 7,5 | 7,5 |
| Ammoniumbicarbonat | 5,0 | 5,0 | 5,0 | 5,0 | 5,0 |
| Diammoniumhydrogencitrat | 30,0 | 30,0 | 30,0 | 30,0 | 30,0 |
| Natriummetasilikat | 11,0 | 11,0 | 11,0 | 11,0 | 11,0 |
| Natriumcarbonat | 11,0 | 11,0 | 11,0 | 11,0 | 11,0 |
| Kaliumcitrat | 10,0 | 10,0 | 10,0 | 10,0 | 10,0 |
| Polyorganosiloxan (Dimethicone) | 1,0 | 0,5 | - | - | - |
| Polyorganosiloxan (Dimethicone-Copolyol) | - | - | 1,0 | 0,5 | - |

Jeweils 20 g der erhaltenen Produkte wurden mit jeweils 40 ml 6%iger wäßriger H₂O₂-Lösung vermischt und die erhaltene Mischung bei 40 °C 30 Minuten auf menschliche Haarsträhnen aufgebracht.

Danach wurden die Strähnen gewaschen, getrocknet und die erreichte Aufhellung mittels der in Beispiel 2 beschriebenen Meßmethode verglichen, wobei folgende Resultate erzielt wurden:

| Produkt | Aufhellung "L*" |
|---|---|
| 3A | 58,79 |
| 3B | 55,49 |
| 3C | 56,37 |
| 3D | 54,93 |
| 3E | 48,91 |

Diese Ergebnisse zeigen, daß auch bei nicht persulfathaltigen Blondierpulvern durch den Zusatz von Polyorganosiloxanen eine signifikant verbesserte Aufhellung erzielt wird.

## Patentansprüche

1. Verwendung von 0,01 bis 2,5 Gew.-%, berechnet auf die Gesamtzusammensetzung, mindestens eines Polyorganosiloxans, zur Verbesserung der Aufhellungswirkung in pulverförmigen Mitteln zum Blondieren von menschlichen Haaren, enthaltend mindestens eine aufhellend wirkende Substanz.

2. Verwendung nach Anspruch 1, gekennzeichnet durch einen Gehalt an 0,05 bis 1 Gew.-%, berechnet auf die Gesamtzusammensetzung, mindestens eines Polyorganosiloxans.

3. Verwendung nach Anspruch 1 oder 2, dadurch gekennzeichnet, daß das Polyorganosiloxan ein Molekulargewicht von etwa 1000 bis etwa 200 000 aufweist.

4. Verwendung nach Anspruch 3, dadurch gekennzeichnet, daß das Polyorganosiloxan ein Molekulargewicht von etwa 3000 bis etwa 100 000 aufweist.

5. Verwendung nach Anspruch 3 oder 4, dadurch gekennzeichnet, daß das Polyorganosiloxan ein Polydimethylsiloxan ist.

6. Verwendung nach Anspruch 3 oder 4, dadurch gekennzeichnet, daß das Polyorganosiloxan ein Poyldimethylsiloxan-Copolyol ist.

## Claims

1. Use of 0,01 to 2,5 % by weight, calculated to the total composition, of at least one organic polysiloxane to enhance the brightening effect of pulverulent compositions for bleaching of human hair comprising at least one effective brightening compound.

2. Use according to claim 1, characterized in that it contains 0.05 to 1% by wt., calculated to the total composition, of at least one organic polysiloxane.

3. Use according to claim 1 or 2, characterized in that the organic polysiloxane has a molecular weight from about 1,000 to about 200, 000.

4. Use according to claim 3, characterized in that the organic polysiloxane has a molecular weight from about 3,000 to about 100,000.

5. Use according to claim 3 or 4, characterized in that it comprises as organic polysiloxane a polydimethyl siloxane.

6. Use according to claim 3 or 4, characterized in that it comprises as organic polysiloxane a polydimethyl siloxane copolyol.

## Revendications

1. Utilisation de 0,01 à 2,5 % en poids, calculé par rapport à la composition totale, d'au moins un polysiloxane organique pour améliorer l'effet d'éclaircissement dans des agents sous forme de poudre pour blondir les cheveux humains, contenant au moins une substance à effet éclaircissant.

2. Utilisation selon la revendication 1, caractérisée par une teneur de 0,05 à 1 % en poids, calculée par rapport à la composition totale, d'au moins un polysiloxane organique.

3. Utilisation selon la revendication 1 ou 2, caractérisée en ce que le polysiloxane organique présente une masse moléculaire d'environ 1 000 à 200 000.

4. Utilisation selon la revendication 3, caractérisée en ce que le polysiloxane organique présente une masse moléculaire d'environ 3 000 à 100 000.

5. Utilisation selon la revendication 3 ou 4, caractérisée en ce que le polysiloxane organique est un polydiméthylsiloxane.

6. Utilisation selon la revendication 3 ou 4, caractérisée en ce que le polysiloxane organique est un polydiméthylsiloxane-co-polyol.
